# DEMANDE DE BREVET EUROPEEN

(11) **EP 1 319 391 A1**
(43) Date de publication de la demande: **18.06.2003**
(21) Numéro de dépôt: 03290488.0
(22) Date de dépôt: 28.08.2000
(51) Int. Cl.: A61K 7/11, A61K 7/06

(54) **Composition cosmétique comprenant au moins un copolymère silicone/acrylate et au moins un agent de conditionnement**

(30) Priorité: 16.09.1999 FR 9911590
(62) Demande divisionnaire de: 00402373.5
(71) Demandeur: L'OREAL, 75008 Paris (FR)
(72) Inventeur: Dupuis, Christine, 75018 Paris (FR)
(74) Mandataire: Bourdeau, Françoise

(57) **Abrégé**

L'invention a pour objet une composition cosmétique, comprenant au moins un copolymère siliconelacrylate particulier et au moins un agent de conditionnement choisi dans le groupe comprenant les polymères cationiques, les tensioactifs cationiques ou amphiphiles, les hydrocarbures linéaires ou ramifiés, les alcools gras, les esters gras et leurs mélanges. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien-et/ou à la mise en forme de la coiffure.

## Description

L'invention a pour objet une composition cosmétique, comprenant au moins un copolymère silicone/acrylate particulier et au moins un agent de conditionnement. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.

Parmi les produits capillaires, notamment ceux destinés à la mise en forme et/ou au maintien de la coiffure les plus répandus sur le marché de la cosmétique, on distingue les compositions à pulvériser essentiellement constituées d'une solution le plus souvent alcoolique ou aqueuse et d'un ou plusieurs matériaux.

Lorsque les compositions sont destinées à la fixation et/ou au maintien de la coiffure, ces matériaux sont généralement des résines polymères, dont la fonction est de former des soudures entre les cheveux, appelés encore matériaux fixants, en mélange avec divers adjuvants cosmétiques. Ces compositions sont généralement conditionnées soit dans un récipient aérosol approprié mis sous pression à l'aide d'un propulseur, soit dans un flacon pompe.

On connaît également les gels ou les mousses capillaires qui sont généralement appliqués sur les cheveux mouillés avant de faire un brushing ou une mise en plis. Pour mettre en forme et fixer la coiffure, on effectue ensuite un brushing ou un séchage. Ces gels ou mousses peuvent également contenir des résines polymères.

Toutefois, ces compositions capillaires présentent souvent l'inconvénient d'altérer les propriétés cosmétiques des cheveux. Ainsi, les cheveux peuvent devenir rêches, difficiles à démêler, perdre leur toucher et leur aspect agréables ou encore manquer de corps. On recherche donc des compositions coiffantes procurant de bonnes propriétés cosmétiques, notamment en terme de démêlage, de douceur et de toucher.

En outre, ces compositions capillaires présentent également l'inconvénient majeur de donner lieu à une effet de poudrage. Au sens de la présente invention, on entend par « poudrage », l'aptitude du matériau obtenu par séchage de la composition capillaire à former une poudre après son application sur les cheveux. Bien entendu, la poudre tombe sur les épaules ou les vêtements de l'utilisateur, ou encore elle accroche au peigne ou à la brosse, et ceci est préjudiciable.

Il existe donc un besoin de trouver des compositions cosmétiques, notamment pour le coiffage, qui ne présentent pas l'ensemble des inconvénients indiqués ci-dessus, et qui en particulier fixent bien la coiffure, tout en procurant de bonnes propriétés cosmétiques, ceci sans effet de poudrage.

De manière surprenante et inattendue, la Demanderesse a découvert que lorsque l'on associe des copolymères silicone/acrylate particuliers avec certains agents de conditionnement, il est possible d'obtenir des compositions cosmétiques répondant aux exigences exprimées ci-dessus.

L'invention a pour objet une composition cosmétique comprenant, dans un milieu cosmétiquement acceptable, au moins un copolymère silicone/acrylate et au moins un agent de conditionnement,
- le copolymère silicone/acrylate étant obtenu par polymérisation radicalaire d'au moins un monomère éthyléniquement insaturé (a) en présence d'au moins un dérivé siliconé (b) comprenant des groupements oxyalkylénés, et
- l'agent de conditionnement étant choisi dans le groupe comprenant les polymères cationiques, les tensioactifs cationiques ou amphiphiles, les organosiloxanes, les hydrocarbures linéaires ou ramifiés en C₈ à C₃₀₀, les alcools gras linéaires ou ramifiés en C₈ à C₃₀, les esters d'acide gras en C₈ à C₃₀ et d'alcool en C₁ à C₃₀, les esters d'acide ou de diacide en C₁ à C₇ et d'alcool gras en C₈ à C₃₀, les céramides ou analogues de céramides.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition pour la fabrication d'une formulation cosmétique capillaire, destinée notamment au maintien et/ou à la mise en forme de la coiffure.

Les copolymères silicone/acrylate particulièrement visés par la présente invention sont ceux décrits dans la demande internationale WO99/04750. En particulier, on préfère le copolymère commercialisé par BASF sous la dénomination Luviflex Silk. Ce polymère un copolymère greffé acrylate de tertiobutyle/ acide méthacrylique et silicone copolyol.

De préférence, le monomère (a) répond à la formule (Iₐ) : 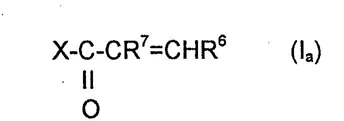 dans laquelle :
- X est choisi dans le groupe comprenant OH, OM, OR⁸, NH₂, NHR⁸, N(R⁸)₂ ;
- M est un cation choisi parmi Na⁺, K⁺, Mg⁺⁺, NH ⁴⁺ , alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

En particulier, les monomères (a) de formule (la) sont l'acide acrylique et ses sels, esters ou amides. Les esters peuvent être dérivés d'alkyles linéaires en C₁ à C₄₀, d'alkyles ramifiés en C₃ à C₄₀ ou d'alcools carboxyliques en C₃ à C₄₀, d'alcools polyfonctionnels ayant 2 à 8 hydroxyles, tel que l'éthylène glycol, l'hexylène glycol, le glycérol et le 1,2,6-hexanetriol, d'aminoalcools ou d'éthers d'alcools tels que le méthoxyméthanol et l'éthoxyéthanol ou les polyalkylènes glycols.

Les monomères (a) de formule (la) peuvent également être choisis parmi les N,N-dialkylaminoalkyl acrylates et méthacrylates et N-dialkylaminoalkylacryl- et -méthacrylamides, le groupement amide pouvant être non substitué, N-aklyl- ou N-alkylamino-monosubstitué ou N,N-dialkylamino-disubstitué, les groupes alkyles ou alkylamino étant dérivés d'unités carboxyliques linéaires en C₁ à C₄₀ ou ramifiées en C₃ à C₄₀.

Les monomères (a) de formule (la) pouvant être utilisés peuvent aussi être des composés substitués dérivés de l'acide acrylique ou ses sels, esters ou amides. On peut citer par exemple les acides méthacrylique, éthacrylique et 3-cyanoacrylique.

D'autres monomères (a) convenant particulièrement bien sont les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

Comme autre monomère (a), on peut enfin citer les N-vinylimidazoles, les diallylamines, le chlorure de vinylidène, les composés insaturés carbonés comme le styrène et l'isoprène, les dérivés quaternisés par l'épichlorohydrine de l'acide acrylique ou méthacrylique.

On préfère tout particulièrement, comme monomère (a) l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

Le monomère (a) peut contenir également des atomes de silicium, de fluor ou encore des groupements thio. Les monomères (a) peuvent être neutralisés s'ils contiennent des groupement acides, et ceci avant ou après la polymérisation, totalement ou partiellement, de façon à ajuster la solubilité ou le degré de dispersion dans l'eau au niveau désiré. En tant qu'agent servant pour la neutralisation, on peut utiliser les bases minérales, telles que le carbonate de sodium, les base organiques telles que les aminoalcools, comme les alcanolamines telles que la méthanolamine, le 2-amino-2-méthyl-1-propanol, la triéthanolamine et les diamines, comme la lysine.

Les monomères (a) peuvent être aussi quaternisés lorsquils possèdent un atome d'azote basique. S'ils possèdent au moins deux doubles liaisons éthyléniques, les monomères (a) peuvent être partiellement réticulés.

Les dérivés siliconés (b) sont notamment les composés connus, sous la dénomination INCI par diméthicone copolyols ou les tensioactifs siliconés. On peut citer ceux commercialisés sous la marque Abil ® par Goldschmidt, Alkasil ® par Rhône-Poulenc, silicone Polyol Copolymer ® par Genesee, Besil ® par Wacker, Silwet ® par OSI ou Dow Corning 190® par Dow Corning.

Parmi les monomères (b), on préfère ceux qui présentent la formule I ci-après : 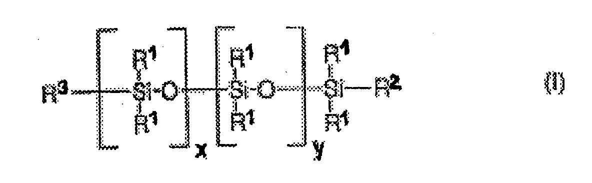 dans laquelle formule (I):
- R² est choisi parmi CH₃ ou le groupement 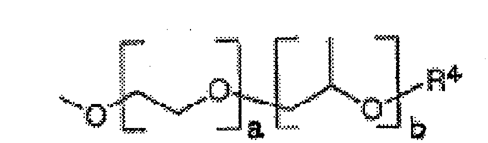
- R³ est choisi parmi CH₃ ou le groupement R²,
- R⁴ est choisi parmi l'hydrogène, CH₃, ou les groupements 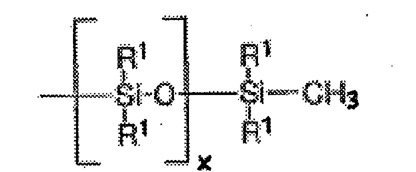 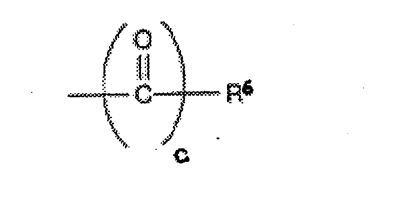
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après :

Parmi les dérivés siliconés (b), on préfère ceux qui présentent la formule I ci-après : 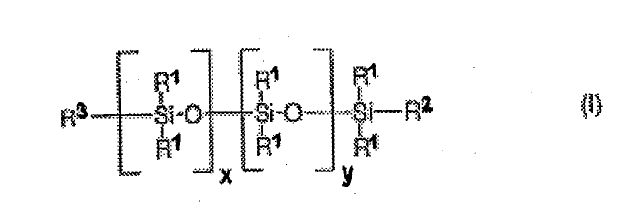 dans laquelle formule (I):
- R² est choisi parmi CH₃ ou le groupement 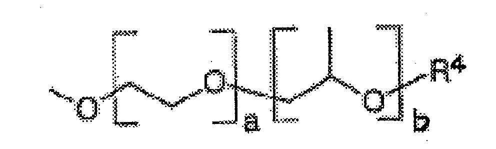
- R³ est choisi parmi CH₃ ou le groupement R²,
- R⁴ est choisi parmi l'hydrogène, CH₃, ou les groupements 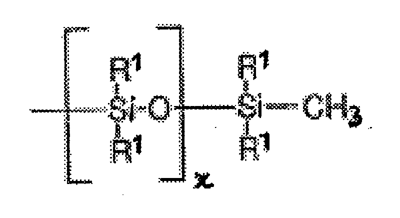 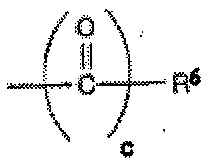
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 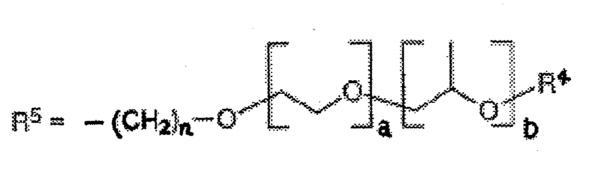
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

De préférence, R¹ est choisi parmi les groupements méthyl, éthyl, propyl, butyl, isobutyl, pentyl, isopentyl, hexyl, octyl, décyl, dodécyl and octadécyl, les radicaux cycloaliphatiques, en particulier les groupements cyclohexyls, les groupements aromatiques, en particulier les groupements phényls ou naphthyls, les mélanges de radicaux aromatiques et aliphatiques, tels que le benzyl et le phenyléthyl, et aussi le tolyl and le xylyl.

On préfère les radicaux R⁴ ayant pour formule -(CO)c-R⁶, R⁶ étant un alkyl, cycloalkyl or aryl ayant 1 à 40 atomes de carbone qui peut contenir des groupements supplémentaires tels que NH₂, COOH et/ou SO₃H.

On préfère les radicaux R⁶ pour lesquels c=0 qui sont des phosphates ou des sulfates.

Les dérivés siliconés (b) particulièrement préférés sont ceux présentant la formule générale suivante :

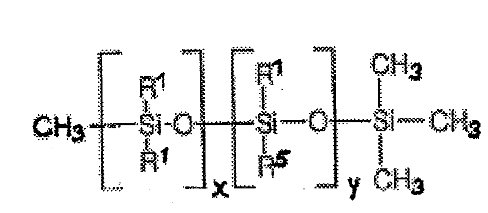

La proportion relative de dérivé (b) dans le copolymère est généralement de 0,1 à 50, et de préférence de 1 à 20 % en poids.

On préfère les copolymères silicone/acrylate solubles dans l'eau ou ceux dont la dispersibilité dans l'eau est telle que dans un mélange eau/éthanol dosé à 50 :50 en volume, ils sont solubles dans une proportion supérieure à 0,1 g/l, et de préférence supérieure à 0,2 g/l.

La composition comprend avantageusement, en pourcentage relatif en poids de la composition, de 0,1 à 20 % de copolymère silicone/acrylate, et plus préférentiellement de 0,5 à 10 % de ce copolymère.

La composition comprend avantageusement, en pourcentage relatif en poids de la composition, de 0,01 à 20 % d'agent de conditionnement et de préférence, de 0,05 à 10 %.

On peut choisir avantageusement un agent de conditionnement insoluble dans le groupe comprenant les poly-α-oléfines, les huiles fluorées, les huiles végétales, les cires naturelles, les cires fluorées, les gommes fluorées et les esters d'acides gras, les silicones insolubles, les composés amides comportant au moins une chaîne grasse ; lesdits agents pouvant être présents sous forme de mélanges.

Les polymères cationiques utilisables comme agents de conditionnement selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Lorsque l'agent de conditionnement est un polymère cationique, il est avantageusement choisi dans le groupe formé par :
(a) les dérivés cationiques cellulosiques ;
(b) les homopolymères et les copolymères d'halogénure de diméthyldiallylammonium;
(c) les homopolymères et copolymères d'halogénure de méthacryloyloxy et éthyltriméthylammonium ;
(d) les polymères polyammonium quaternaire ;
(e) les copolymères de vinylpyrrolidone à motifs cationi ques ;
(f) les polysiloxanes cationiques ;
(g) les polysaccharides cationiques ;
(h) les polyamidoamines.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 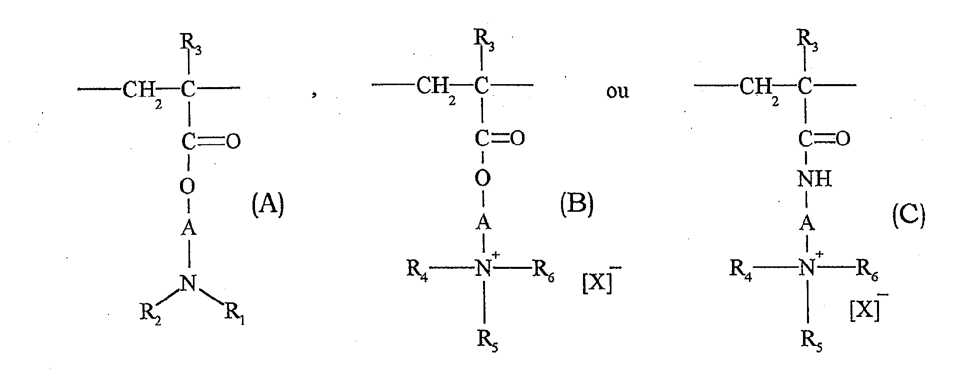 dans lesquelles:
   R₃ désigne H ou CH₃;
   A est un groupe alkyle linéaire ou ramifié de 1 à 6 atomes de carbone ou un groupe hydroxyalkyle de 1 à 4 atomes de carbone ;
   R4, R5, R6, identiques ou différents, représentent un groupe alkyle ayant de 1 à 18 atomes de carbone ou un radical benzyle;
   R1 et R2 représentent hydrogène ou un groupe alkyle ayant de 1 à 6 atomes de carbone;
   X désigne un anion méthosulfate ou un halogénure tel que chlorure ou bromure.

   Les copolymères contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone, des esters vinyliques.
   Parmi ces composés, on peut citer :
   - le copolymère d'acrylamide et de diméthylaminoéthyl méthacrylate quaternisé au sulfate de diméthyle vendu sous la dénomination HERCOFLOC par la société HERCULES,
   - le copolymère d'acrylamide et de chlorure de méthacryloyloxyéthyl-triméthylammonium décrit dans la demande de brevet EP-A-080976 et vendu sous la dénomination BINA QUAT P 100 par la société CIBA GEIGY,
   - le copolymère d'acrylamide et de méthosulfate de méthacryloyloxyéthyl-triméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - et le copolymère vinylpyrrolidone / méthacrylamide de diméthylaminopropyle quaternisé tel que le produit vendu sous la dénomination "GAFQUAT HS 100" par la société ISP.
(2) les polysaccharides quaternisés décrits plus particulièrement dans les brevets américains 3.589.578 et 4.031.307 tel que le produit commercialisé sous la dénomination JAGUAR C 13 S par la société MEYHALL;
(3) les copolymères quaternaires de vinylpyrrolidone et de vinylimidazole, tels que les produits commercialisés sous la dénomination générale Luviquat par la Société Basf ;

Selon l'invention, on met de préférence en oeuvre, des polymères cationiques présentant des propriétés filmogènes.

On peut choisir avantageusement des agents tensioactifs cationiques insolubles dans l'eau dans le groupe constitué par les amines grasses et leurs sels ainsi que les sels d'ammonium quaternaire. De préférence, les amines grasses sont choisies dans le groupe comprenant la dioctylamine, la stéaryldiméthylamine, la palmityl-diméthylamine, l'oléocétyldiméthylamine et les amidoamines telles que la stéarylamido-éthyldiéthylamine, la béhénylamidopropyldiméthylamine, la stéarylamidopropyldiméthyl-amine, l'oléylamido-propyldiméthylamine, la stéarylamidoéthyldiméthylamine.

L'agent tensioactif cationique de type sel d'ammonium quaternaire est choisi avantageusement parmi ceux qui présentent la formule générale (I) suivante : 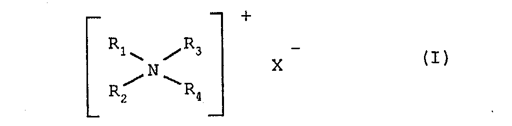 dans laquelle R₁ à R₄, identiques ou différents, représentent un radical aliphatique comportant de 1 à 22 atomes de carbone, ou un radical aromatique, alcoxy, polyoxyalkylène, alkylamide, hydroxyalkyle, aryle ou alkylaryle comportant de 12 à 22 atomes de carbone; X est un anion choisi dans le groupe des halogénures, phosphates, acétates, lactates ou alkylsulfates,
ou les sels d'ammonium quaternaire de l'imidazolinium, de formule (II) suivante : 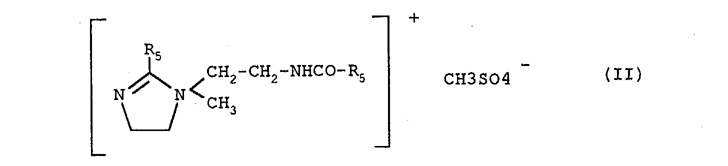 dans laquelle R₅ représente un mélange de radicaux alcényle et/ou alkyle comportant de 13 à 21 atomes de carbone et dérivés des acides gras du suif,
ou les sels de diammonium quaternaire de formule (III) : 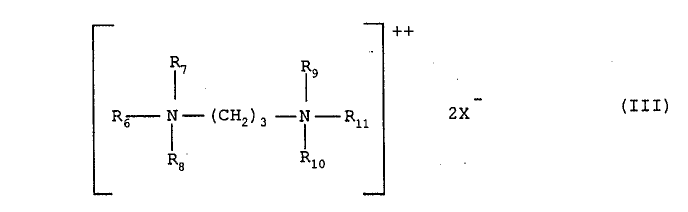 dans laquelle R₆ désigne un radical aliphatique comportant de 16 à 22 atomes de carbone, R₇, R₈, R₉, R₁₀, et R₁₁ sont choisis parmi l'hydrogène ou un radical alkyle comportant de 1 à 4 atomes de carbone, et X est un anion choisi dans le groupe des halogénures, acétates, phosphates, et sulfates.

Parmi les organosiloxanes utilisés conformément à la présente invention, on peut citer à titre non limitatif :

### I. Les silicones volatiles

Celles-ci possèdent un point d'ébullition compris entre 60°C et 260°C. Parmi ce type de silicones, on cite :
(i) les silicones cycliques de 3 à 7 atomes de silicium et de préférence 4 à 5. Il s'agit, par exemple, de l'octaméthylcyclo-tétrasiloxane vendu sous le nom de "VOLATILE SILICONE 7207®" par la Société UNION CARBIDE ou "SILBIONE 70045 V2®" par la Société RHONE POULENC, le décaméthylcyclopentasiloxane vendu sous le nom de "VOLATILE SILICONE 7158®" par la Société UNION CARBIDE, "SILBIONE 70045 V5®" par la Société RHONE POULENC, ainsi que leurs mélanges.
   On cite également les cyclopolymères du type diméthylsiloxane/méthylalkylsiloxane, tel que la "SILICONE VOLATILE FZ 3109®" vendue par la Société UNION CARBIDE, qui est un cyclocopolymère diméthylsiloxane/méthyloctylsiloxane ;
(ii) les silicones volatiles linéaires ayant 2 à 9 atomes de silicium et possédant une viscosité inférieure ou égale à 5.10⁻⁶ m²/s à 25°C. Il s'agit, par exemple, de l'hexaméthyldisiloxane vendu sous la dénomination "SILBIONE 70041 V0,65®" par la Société RHONE POULENC. Ce type de produit est décrit dans l'article de TODD & BYERS "Volatile silicone fluides for cosmetics", Cosmetics and Toiletries, Vol. 91, Jan 76, pages 27-32.

### II. Les silicones non volatiles

Elles sont constituées principalement par les polyalkyl (C1-C35)-siloxanes, les polyarylsiloxanes, les polyalkyl (C1-C35)arylsiloxanes, les gommes et résines de silicone et les polysiloxanes organomodifiés, ainsi que leurs mélanges.

Parmi les polyalkylsiloxanes, on peut citer principalement les polydiméthylsiloxanes linéaires de viscosité supérieure à 5.10⁻⁶ m²/s, et de préférence inférieure à 2,6 m²/s soit :
- à groupements terminaux triméthylsilyle, comme par exemple, et à titre non limitatif, les huiles "SILBIONE®" de la série 70047 commercialisées par la Société RHONE POULENC, l'huile "47 V 500.000®" de RHONE POULENC ou certaines "VISCASIL®" de la Société GENERAL ELECTRIC,
- à groupements terminaux trihydroxysilyle, tels que les huiles de la série "48 V®" de la Société RHONE POULENC.

Dans cette classe de polyalkylsiloxanes, on peut également mentionner les polyalkylsiloxanes vendus par la Société GOLDSCHMIDT sous les dénominations "ABILWAX 9800®" et "ABILWAX 9801®", qui sont des polyalkyl(C₁-C₂₀)siloxanes.

Parmi les polyalkylarylsiloxanes, on peut citer les polydiméthylphénylsiloxanes, les polydiméthyldiphénylsiloxanes linéaires et/ou ramifiés, de viscosité 10⁻⁵ à 5.10⁻² m²/s, tels que par exemple :
- l'huile "RHODORSIL®" 763 de RHONE POULENC,
- les huiles "SILBIONE®" de la série 70641 de RHONE POULENC, telles que les huiles "SILBIONE 70641 V30®" et "SILBIONE 70641 V200®" de RHONE POULENC,
- le produit "DC 556®" Cosmetic Grad Fluid de Dow CORNING,
- les silicones de séries PK de BAYER, telles que la "PK20®",
- les silicones des séries PN, PH de BAYER, comme les "PN 1000®" et "PH 1000®",
- certaines huiles des séries SF de GENERAL ELECTRIC, telles que les "SF 1250®", "SF 1265®", "SF 1154®", "SF 1023®".

Les gommes de silicone, conformes à la présente invention, sont des polydiorganosiloxanes de forte masse moléculaire moyenne en nombre comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On cite, par exemple, les composés ayant les structures suivantes:
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)].

On peut citer, par exemple, à titre non limitatif, les mélanges suivants :
1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature-CTFA), tels que le produit "Q2 1401®" vendu par la Société Dow CORNING ;
2) les mélanges formés à partir d'une gomme polydiméthylsiloxane avec une silicone cyclique, tel que le produit "SF 1214 SILICONE FLUID®" de GENERAL ELECTRIC, qui est une gomme "SE 30®" de PM 500.000 (Mn) solubilisée dans la "SF 1202 SILICONE FLUID®" (décaméthylcyclopentasiloxane) ;
3) les mélanges de deux PDMS de viscosités différentes, notamment d'une gomme PDMS et d'une huile PDMS, tels que les produits "SF 1236®" et "CF 1241®" de la Société GENERAL ELECTRIC. Le produit "SF 1236®" est le mélange d'une gomme "SE 30®" définie ci-dessus d'une viscosité de 20 m²/s et d'une huile "SF 96®" d'une viscosité de 5.10⁻⁶ m²/s (15 % de gomme "SE 30®" et 85 % d'huile "SF 96®").

Le produit "CF 1241®" est le mélange d'une gomme "SE 30®" (33 %) et d'une PDMS (67 %) de viscosité 10⁻³ m²/s.

Les résines d'organopolysiloxanes utilisables conformément à l'invention, sont des systèmes siloxaniques réticulés renfermant les unités: R₂SiO_{2/2}, RSiO_{3/2} et SiO_{4/2} dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination "DOW CORNING 593®" ou ceux vendus sous les dénominations "SILICONE FLUID SS 4230" et "SILICONE FLUID SS 4267" par la Société GENERAL ELECTRIC et qui sont des diméthyl/triméthylpolysiloxanes.

Les silicones organomodifiées, conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les silicones comportant :
a) des groupements perfluorés tels que des trifluoroalkyles comme, par exemple, celles vendues par la Société GENERAL ELECTRIC sous les dénominations "FF.150 FLUOROSILICONE FLUID®" ou par la Société SHIN ETSU sous les dénominations "X-22-819®", "X-22-82®", "X-22-821®" et "X-22-822®" ;
b) des groupements hydroxyacylamino comme, par exemple, celles décrites dans la demande de brevet EP-A-0 342 834 et en particulier la silicone vendue par la Société Dow CORNING sous la dénomination "Q2-8413®" ;
c) des groupements thiols comme dans les silicones "X 2-8360®" de la Société Dow CORNING ou les "GP 72A®" et "GP 71®" de GENESEE ;
d) des groupements aminés substitués ou non, comme dans, la "GP 4 SILICONE FLUID®" de GENESEE, la "GP 7100®" de GENESEE, la "Q2 8220®" de Dow CORNING, l'''AFL 40®" d'UNION CARBIDE ou la silicone dénommée "Amodiméthicone" dans le dictionnaire CTFA ;
e) les groupements carboxylates, comme les produits décrits dans le brevet EP_186 507 de CHISSO CORPORATION ;
f) des groupements hydroxylés, comme les polyorgano-siloxanes à fonction hydroxyalkyle, décrits dans la demande de brevet FR-85 16334, répondant à la formule suivante : 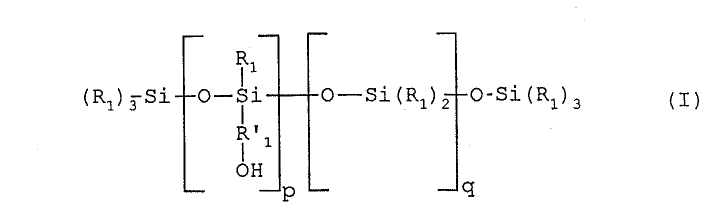 dans laquelle :
   - les radicaux R₁, identiques ou différents, sont choisis parmi les radicaux méthyle et phényle, au moins 60 % en mole des radicaux R₁ étant méthyle ;
   - le radical R'₁ est un chaînon alkylène divalent hydrocarboné en C₂-C₁₈ ;
   - p est compris entre 1 et 30 inclus ;
   - q est compris entre 1 et 150 inclus .
      On peut citer tout particulièrement le produit commercialisé par Dow Corning sous l'appellation DC 190;
g) des groupements alcoxylés comme dans la Silicone copolymer "F 755®" de SWS SILICONES et les produits "ABILWAX 2428®", "ABILWAX 2434®", "ABILWAX 2440®" de la Société GOLDSCHMIDT ;
h) des groupements acyloxyalkyles, comme par exemple les polyorganopolysiloxanes décrits dans la demande de brevet FR-88 17433, répondant à la formule suivante : 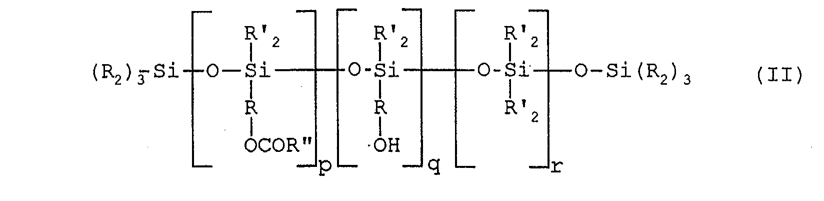 dans laquelle :
   - R₂ désigne méthyle, phényle, OCOR", hydroxyle, un seul des R₂ par atome de silicium peut être OH ;
   - R'₂ désigne méthyle, phényle, 60 % molaire au moins de l'ensemble des radicaux R₂ et R'₂ est méthyle ;
   - R" désigne alcoyle ou alcényle en C₈-C₂₀ ;
   - R désigne un alkylène hydrocarboné divalent, linéaire ou ramifié en C₂-C₁₈ ;
   - r est compris entre 1 et 120 inclus ;
   - p est compris entre 1 et 30 inclus ;
   - q vaut 0 ou est inférieur à 0,5 p, p + q étant compris entre 1 et 30 inclus :
   les polyorganosiloxanes de formule (II) peuvent contenir des groupements 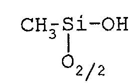 dans des proportions ne dépassant pas 15 % de la somme p + q + r ;
i) des groupements ammonium quaternaires, comme dans les produits "X2 81 08®" et "X2 81 09®", le produit "ABIL K 3270®" de la Société GOLDSCHMIDT ;
j) des groupements amphotères ou bétaïniques, tels que dans le produit vendu par la Société GOLDSCHMIDT sous la dénomination "ABIL B 9950®" ;
k) des groupements bisulfites, tels que dans les produits vendus par la Société GOLDSCHMIDT sous les dénominations "ABIL S 201®" et "ABIL S 255®".

Les viscosités des silicones peuvent être notamment déterminées par la norme ASTM D445-97 (viscométrie).

Lorsque l'agent de conditionnement de la composition selon l'invention est un hydrocarbure, celui-ci est un hydrocarbure, linéaire ou ramifié en C₈-C₃₀₀. Parmi les hydrocarbures liquides à température ambiante répondant à cette définition, on peut notamment citer l'isododécane, l'isohexadécane et ses isomères (tels que le 2,2,4,4,6,6-heptaméthylnonane), l'isoeicosane, l'isotétracosane, et les isomères desdits composés. On utilise de préférence selon l'invention l'isododécane ou l'un de ses isomères.

Lorsque l'agent de conditionnement est un alcool gras, celui-ci est du type linéaire ou ramifié, saturé ou insaturé, en C₈-C₃₀ et parmi ceux-ci on peut citer le butyl-2 octanol, l'alcool laurique, l'alcool oléique, l'alcool isocétylique, l'alcool isostéarique et l'alcool béhénique.

Lorsque l'agent de conditionnement est un ester gras, celui-ci peut être soit un ester d'un acide gras en C₈-C₃₀ et d'alcool en C₁-C₃₀ soit un ester d'un acide ou diacide en C₁-C₇ et d'un alcool gras en C₈-C₃₀. Parmi ces esters on peut citer le palmitate d'éthyle, d'isopropyle, d'éthyl-2-hexyle et de 2-octyldécyle, le myristate d'isopropyle, de butyle, de cétyle et de 2-octyldécyle, le stéarate de butyle et d'hexyle, le laurate d'hexyle et de 2-hexyldécyle, l'isononanoate d'isononyle et le malate de dioctyle.

Les céramides ou analogues, tels que les glycocéramides utilisables dans les compositions selon l'invention, sont connus en eux-mêmes et sont des molécules naturelles ou synthétiques pouvant répondre à la formule générale suivante : 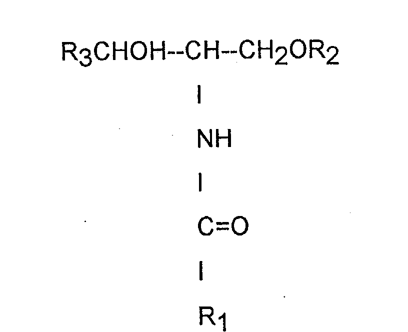 dans laquelle :
- R₁ désigne un radical alkyle, linéaire ou ramifié, saturé ou insaturé, dérivé d'acides gras en C₁₄-C₃₀, ce radical pouvant être substitué par un groupement hydroxyle en position α, ou un groupement hydroxyle en position ω estérifié par un acide gras saturé ou insaturé en C₁₆-C₃₀ ;
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un radical hydrocarboné en C₁₅-C₂₆, saturé ou insaturé en position α, ce radical pouvant être substitué par un ou plusieurs radicaux alkyle en C₁-C₁₄ ;
étant entendu que dans le cas des céramides ou glycocéramides naturelles, R₃ peut également désigner un radical α-hydroxyalkyle en C₁₅-C₂₆, le groupement hydroxyle étant éventuellement estérifié par un α-hydroxyacide en C₁₆-C₃₀.

Les céramides préférées dans le cadre de la présente invention sont celles décrites par DOWNING dans Arch. Dermatol, Vol. 123, 1381-1384, 1987, ou celles décrites dans le brevet français FR-2 673 179, dont les structures peuvent être les suivantes :

Les céramides plus particulièrement préférés selon l'invention sont les composés pour lesquels R₁ désigne un alkyle saturé ou insaturé dérivé d'acides gras en C₁₆-C₂₂ ; R₂ désigne un atome d'hydrogène ; et R₃ désigne un radical linéaire saturé en C₁₅.

De tels composés sont par exemple :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyidihydrosphingosine,
ou les mélanges de ces composés.

Encore plus préférentiellement, on utilise les céramides pour lesquels R₁ désigne un radical alkyle saturé ou insaturé dérivé d'acides gras ; R₂ désigne un radical galactosyle ou sulfogalactosyle ; et R₃ désigne un groupement

-CH=CH-(CH₂)₁₂-CH₃.

A titre d'exemple, on peut citer le produit constitué d'un mélange de ces composés, vendu sous la dénomination commerciale GLYCOCER par la société WAITAKI INTERNATIONAL BIOSCIENCES.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

La composition de l'invention peut également contenir au moins un additif choisi parmi les tensioactifs autres que ceux de l'invention, les parfums, les filtres, les conservateurs, les protéines, les vitamines, les polymères autres que ceux de l'invention, les huiles minérales, végétales ou synthétiques autres que celles de l'invention, les agents épaississants et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'invention peuvent être appliquées sur la peau, les ongles, les lèvres, les cheveux, les sourcils et les cils.

Les compositions conformes à l'invention sont particulièrement adaptées pour être appliquées sur des cheveux secs ou humides, en tant que produit de coiffage.

L'invention va être plus complètement illustrée à l'aide de l'exemple non limitatif suivant.

Tous les pourcentages sont des pourcentages relatifs en poids par rapport au poids total de la composition et m.a. signifie matière active.

### EXEMPLE :

On applique la composition A suivante sur une mèche de cheveux naturels eurochâtain de 5 grammes.

| Composition A : | |
|---|---|
| Luviflex Silk (1) | 6% |
| DC190(2) | 0,6% |
| AMP | qs neutralisation à 100% |
| Ethanol | qsp100% |

| | |
|---|---|
| (1) Copolymère silicone/acrylate commercialisé par BASF (t-butyl acrylate/acide méthacrylique/PDMS polyéther) | |
| (2) Silicone diméthiconol commercialisée par Dow Corning | |

La composition A est appliquée à l'aide d'un flacon pompe sur des cheveux naturels. Après séchage, on observe que la mèche est douce au toucher, d'un aspect agréable et qu'elle poudre peu au peignage.

## Revendications

1. Composition cosmétique comprenant, dans un milieu cosmétiquement acceptable, au moins un copolymère silicone/acrylate et au moins un agent de conditionnement, **caractérisée par le fait que**
- le copolymère silicone/acrylate est obtenu par polymérisation radicalaire d'au moins un monomère éthyléniquement insaturé (a) en présence d'au moins un dérivé siliconé (b) comprenant des groupements oxyalkylénés, et
- l'agent de conditionnement est choisi dans le groupe comprenant les polymères cationiques, les tensioactifs cationiques ou amphiphiles, les hydrocarbures linéaires ou ramifiés en C₈ à C₃₀₀, les alcools gras linéaires ou ramifiés en C₈ à C₃₀, les esters d'acide gras en C₈ à C₃₀ et d'alcool en C₁ à C₃₀, les esters d'acide ou de diacide en C₁ à C₇ et d'alcool gras en C₈ à C₃₀, les céramides ou analogues de céramides,
- les polymères cationiques étant choisis parmi
(a) les dérivés cationiques cellulosiques ;
(b) les homopolymères et les copolymères d'halogénure de diméthyldiallylammonium;
(c) les homopolymères et copolymères d'halogénure de méthacryloyloxy et éthyltriméthylammonium ;
(d) les polymères polyammonium quaternaire ;
(e) les polysiloxanes cationiques ;
(f) les polysaccharides cationiques ;
(g) les polyamidoamines, et

2. Composition cosmétique selon la revendication 1, **caractérisée par le fait que** le monomère (a) répond à la formule (Iₐ) : 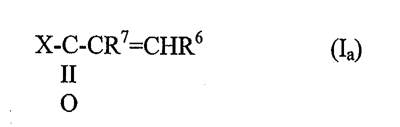 dans laquelle :
- X est choisi-dans le groupe comprenant OH, OM, OR⁸, NH₂, NHR⁸, N(R⁸)₂ ;
- M est un cation choisi parmi Na⁺, K⁺, Mg⁺⁺, NH ⁴⁺ , alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

3. Composition cosmétique selon la revendication 1, **caractérisée par le fait que** le dérivé siliconé (b) répond à la formule I ci-après : 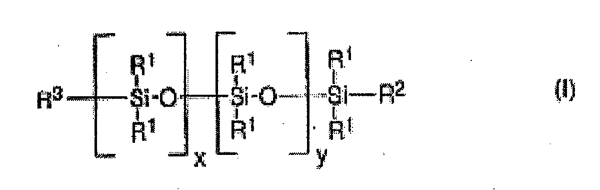 dans laquelle formule (I):
- R² est choisi parmi CH₃ ou le groupement 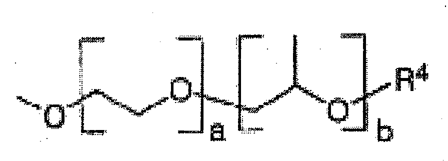
- R³ est choisi parmi CH₃ ou le groupement R²,
- R⁴ est choisi parmi l'hydrogène, CH₃, ou les groupements 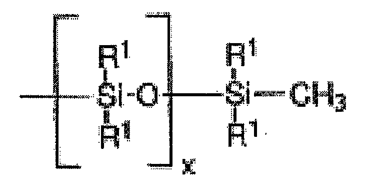 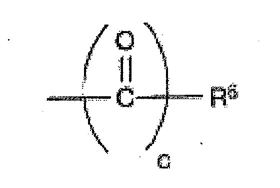
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 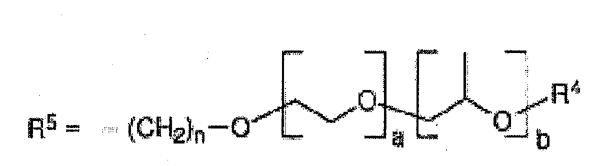
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

4. Composition selon la revendication 1, **caractérisée par le fait que** le monomère (a) est choisi parmi les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

5. Composition selon la revendication 1, **caractérisée par le fait que** le monomère (a) est choisi dans le groupe comprenant l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

6. Composition selon l'une quelconque des revendications précédentes, **caractérisée par le fait que** les dérivés siliconés (b) sont choisis parmi les diméthicone copolyols ou les tensioactifs siliconés.

7. Composition selon l'une quelconque des revendications précédentes, **caractérisée par le fait qu'**elle comprend, en pourcentage relatif en poids, de 0,1 à 20 % de copolymère silicone/acrylate, et plus préférentiellement de 0,5 à 10 % de ce copolymère.

8. Composition selon l'une quelconque des revendications précédentes, **caractérisée par le fait qu'**elle comprend, en pourcentage relatif en poids, de 0,01 à 20 % d'agent de conditionnement et de préférence, de 0,05 à 10 % .

9. Composition selon l'une quelconque des revendications 1 à 8, **caractérisée par le fait que** l'agent de conditionnement est un hydrocarbure linéaire ou ramifié en C₈-C₃₀₀ choisi parmi l'isododécane, l'isohexadécane et ses isomères, l'isoeicosane, l'isotétracosane, et les isomères desdits composés.

10. Composition selon l'une quelconque des revendications 1 à 6, **caractérisée par le fait que** l'agent de conditionnement est un ester gras choisi parmi le palmitate d'éthyle, d'isopropyle, d'éthyl-2-hexyle et de 2-octyldécyle, le myristate d'isopropyle, de butyle, de cétyle et de 2-octyldécyle, le stéarate de butyle et d'hexyle, le laurate d'hexyle et de 2-hexyldécyle, l'isononanoate d'isononyle et le malate de dioctyle.

11. Composition selon la revendication 1, **caractérisée par le fait que** l'agent de conditionnement est une céramide ou analogue et répond à la formule: 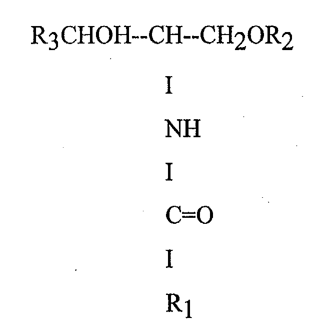 dans laquelle :
- R₁ désigne un radical alkyle, linéaire ou ramifié, saturé ou insaturé, dérivé d'acides gras en C₁₄-C₃₀, ce radical pouvant être substitué par un groupement hydroxyle en position α, ou un groupement hydroxyle en position ω estérifié par un acide gras saturé ou insaturé en C₁₆-C₃₀ ;
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un radical hydrocarboné en C₁₅-C₂₆, saturé ou insaturé en position α, ce radical pouvant être substitué par un ou plusieurs radicaux alkyle en C₁-C₁₄ ;
étant entendu que dans le cas des céramides ou glycocéramides naturelles, R₃ peut également désigner un radical α-hydroxyalkyle en C₁₅-C₂₆, le groupement hydroxyle étant éventuellement estérifié par un α-hydroxyacide en C₁₆-C₃₀.

12. Composition selon l'une quelconque des revendications précédentes, **caractérisée par le fait qu'**elle contient en outre au moins un additif choisi parmi les tensioactifs autres que ceux de l'invention, les parfums, les filtres, les conservateurs, les protéines, les vitamines, les polymères autres que ceux de l'invention, les huiles minérales, végétales ou synthétiques autres que celles de l'invention, les épaississants.

13. Dispositif aérosol formé par un récipient contenant une composition selon l'une quelconque des revendications précédentes et un gaz propulseur, ainsi qu'un moyen de distribution de la composition.

14. Procédé cosmétique, **caractérisé par le fait qu'**il comprend l'application d'une composition conforme à l'une quelconque des revendications 1 à 12, en particulier sur les cheveux.

15. Utilisation d'une composition selon l'une quelconque des revendications 1 à 12, pour la fabrication d'un produit cosmétique, en particulier capillaire.

16. Utilisation d'une composition selon l'une quelconque des revendications 1 à 12 pour les cheveux, les sourcils et les cils.
